# EUROPEAN PATENT SPECIFICATION

(11) **EP 3 305 111 B1**
(45) Date of publication and mention of the grant of the patent: **22.12.2021**
(21) Application number: 18151984.4
(22) Date of filing: 16.01.2018
(51) Int. Cl.: A24F 40/40, A61M 15/00, A61M 15/06, A24F 7/02

(54) **ELECTRONIC CIGARETTE**
ELEKTRONISCHE ZIGARETTE
CIGARETTE ÉLECTRONIQUE

(30) Priority: 25.05.2017 CN 201720590897 U
(43) Date of publication of application: 11.04.2018
(73) Proprietor: Shenzhen First Union Technology Co., Ltd., Shenzhen, Guangdong 518104 (CN)
(72) Inventor: LI, Yonghai, Shenzhen, Guangdong 518104 (CN); XU, Zhongli, Shenzhen, Guangdong 518104 (CN); OUYANG, Zhi, Shenzhen, Guangdong 518104 (CN); LI, Meng, Shenzhen, Guangdong 518104 (CN); CHEN, Huiyun, Shenzhen, Guangdong 518104 (CN)
(74) Representative: Proi World Intellectual Property GmbH

(56) References cited:
- EP-A1- 2 022 349
- WO-A1-2013/118299
- JP-A- 2012 034 666

## Description

### TECHNICAL FIELD

The present disclosure relates to the field ofelectronic cigarettes, and particularly, to an electronic cigarette having an invertible mouthpiece.

### BACKGROUND

Tobacco smoke contains dozens of carcinogens (for example, tar), which are believed to have a great harm to human health. Furthermore, thesmoke hangs in air, making the surrounding people passive to smoke and causingdamages to their bodies. Therefore, most public places forbid smoking expressly. In order to meet the requirements of part smokers, electronic cigarette appears accordingly.

At present, most electronic cigarettes available on the market have the mouthpiece exposed to outside. The mouthpiece is easy to be contaminated by dust, oil, etc., thereby influencing the experience of consumers. A few electronic cigarettes can be put in a storage box or accommodation box entirely after use. However, configuring a storage box or accommodation box separately increases the cost, and sometimes is inconvenient.

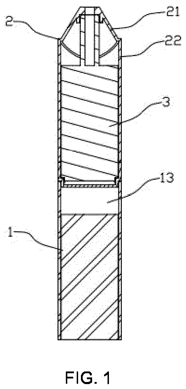

For example, a Chinese patent with application number of CN201620403775.8 discloses a mouthpiece and an electronic cigarette thereof, in which the mouthpiece can be stored in a storage groovein a rotatable manner, so that the external contamination to the mouthpiece is reduced. As shown in FIG. 1, after the mouthpiece is stored, the inhaling end is still partially exposed to outside.It's not possible to completely avoid contamination. Another Chinese patent with application number of CN201420654658.X discloses a flue-cured type electronic cigarette having a telescopic mouthpiece, in which, one end of a guide rail is connected to a mouthpiece, and a housing defines therein a movement groove allowing the guide rail to move up down. Such arrangement can avoid the condition that the mouthpiece is over protruded from the surface of the electronic cigarette during the non-use state. However, this scheme also cannot completely prevent the mouthpiece being contaminated.
EP2022349A1 discloses an aerosol electronic cigarette.

### SUMMARY

The technical problem to be solved by the present disclosure is to remedy the drawbacks of the prior art by providing an electronic cigarette which is capable of completely hiding a mouthpiece, and is convenient to operate and low in cost.

In order to solve the above technical problem, the present disclosure employs a following technical scheme. The present disclosure provides an electronic cigarette according to independent claim 1. Various improvements to the electronic cigarette are recited in the dependent claims. An electronic cigarette having an invertible mouthpiece includes a battery assembly and an atomization assembly, the battery assembly including a battery and a housing, and the atomization assembly including a mouthpiece, an outer sleeve and a cartridge. The atomization assembly and the battery assembly are in pluggable connection. The housing defines a groove in the upper part thereof. The outer sleeve has a tail end accommodated into the groove in a working state, and the atomization assembly is inverted and the mouthpiece is accommodated into the groove in a receiving state.The cartridge and the outer sleeve are in a detachable structure.The outer sleeve defines a longitudinalslot on a lateral edge thereof, the cartridge is provided with a convex column on the outside, the position of the convex column corresponds to position of the slot, and the convex column protrudes from the outer surface of the outer sleeve.

The outer sleeve has an outer surface matched with the housing in shape and size.

The mouthpiece and the outer sleeve of the atomization assembly are in an integrated structure or detachable structure.

The number of the slot is two, and the number of the convex column is two too.

The outer sleeve is provided with a bottom cap; the bottom cap and the mouthpiece are disposed at two ends of the outer sleeve respectively.

The outer sleeve defines a longitudinal vision window.

The beneficial effects of the present disclosure are as follows. The atomization assembly and the battery assembly are in pluggable connection,thus it is convenient and labor saving to invert the mouthpiece. In each of the working state and the receiving state, the outer surface of the outer sleeve fully fits the upper part of the housing, thereby achieving a general dustproof effect, saving space for the user and protecting the user's privacy properly.

### BRIEF DESCRIPTION OF THE DRAWINGS

FIG. 1 is a cross-sectional view of an electronic cigarette in a working state.
FIG. 2 is a cross-sectional view of an electronic cigarettein a receiving state.
FIG. 3 is a perspective view of an electronic cigarette in a working state.
FIG. 4 is a perspective view of an electronic cigarettein a receiving state.
FIG. 5 is a side view of an electronic cigarette in a working state.
FIG. 6 is a side view of an electronic cigarette in an intermediate state.
FIG. 7 is a side view of an electronic cigarettein a receiving state.
FIG. 8 is a perspective view of a second embodiment of an electronic cigarette in a working state.
FIG. 9 is a perspective view of a second embodiment of an electronic cigarettein a receiving state.
FIG. 10 is an exploded view of a second embodiment of an electronic cigarettein a receiving state.
FIG. 11 is a cross-sectional view of a third embodiment of an electronic cigarettein a working state.
FIG. 12 is an exploded view of a third embodiment of an electronic cigarette in a working state.
FIG. 13 is an exploded view of a fourth embodiment of an electronic cigarette in a working state.

### DETAILED DESCRIPTION

An electronic cigarette having an invertible mouthpiece provided by the present disclosure is described below in further detail in conjunction with the drawings.

FIG. 1 to FIG. 7 illustrates a first embodiment of an electronic cigarette. The electronic cigarette includes a battery assembly 1 and an atomization assembly 2 that are coupled together. The battery assembly 1 includes a housing 11, and a battery 12 built in the housing. The atomization assembly 2 includes a mouthpiece 21 and an outer sleeve 22. The mouthpiece 21 is located at an upper end of outer sleeve 22.

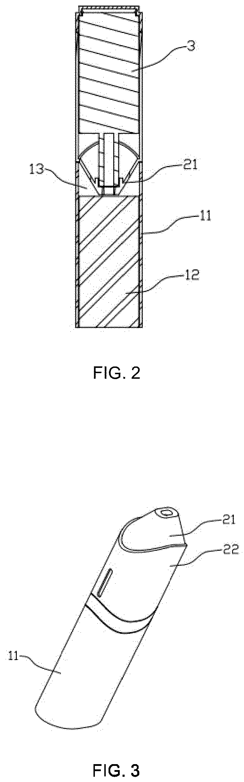

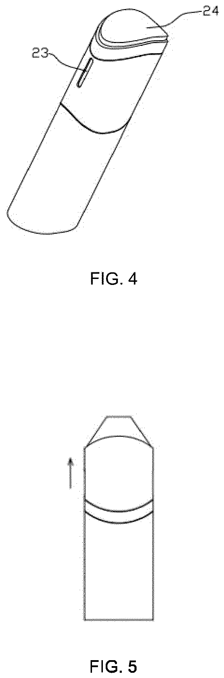

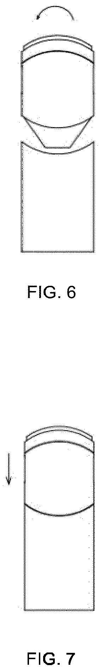

The housing 11 of the battery assembly 1 defines a groove 13 in the upper part thereof. In a working state, as shown in FIG. 1, FIG. 3 and FIG. 5, the outer sleeve 22 has a tail end accommodated into the groove 13. The outer sleeve 22 has an outer surface matched with the housing 11 in shape and size. After the electronic cigarette is used, during the storage process, as shown in FIG. 5, the atomization assembly 2 is lifted upwards and pulled out entirely, so that the tail end of the atomization assembly 2 is disengaged from the groove 13; then, as shown in FIG. 6, the atomization assembly 2 is overturned downwards by 180 degrees entirely; next, as shown in FIG. 7, the atomization assembly 2 is inserted downwards, so that the mouthpiece 21 of the atomization assembly 2 is completely accommodated into the groove 13. The working state is as shown in FIG. 3, and the receiving state is as shown in FIG. 4. The outer surface of the outer sleeve 22 fully fits the upper part of the housing 11, thereby being capable of preventing entrance of water and dust. In this way, the mouthpiece 21 is stored by simple and quick pullout and insertion, without extra parts. This arrangement not only achieves a general dustproof effect, but also saves space for the user and protects the user's privacy properly. When the atomization assembly 2 is operated from the receiving state to the working state, the operation is just the opposite, and no further description is needed here.

In the first embodiment, the atomization assembly 2 further has a cartridge 3 disposed therein. The cartridge 3 can contain tobacco liquid, tobacco paste, solid tobacco material, etc. The cartridge 3 and the outer sleeve 22 are fixed through an integrated structure. The mouthpiece 21 and the outer sleeve 22 are in an integrated structure, that is, the mouthpiece 21 and the outer sleeve 22 are not detachable from each other. The outer sleeve 22 further defines a vision window 23. The vision window 23 is defined longitudinal, so that the user can check the rest of the cartridge 3 at any time. The outer sleeve 22 is further provided with a bottom cap 24. The bottom cap 24 and the mouthpiece 21 are disposed at two opposite ends of the outer sleeve 22 respectively.

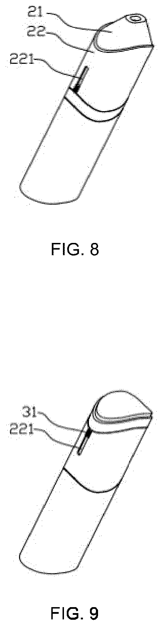

A second embodiment of the electronic cigarette is as shown in FIG. 8 to FIG. 10. The present embodiment has most of the structure the same as the first embodiment, with a difference lying that the cartridge 3 and the outer sleeve 22 are in a detachable structure. Such arrangement has the purposes that an exhausted cartridge 3 can be replaced and that the outer sleeve 22 and the mouthpiece 21 can be reused to save consumption of materials. In the second embodiment, the outer sleeve 22 defines a longitudinalslot 221 on a lateral edge thereof, the cartridge 3 is provided with a convex column 31 on the outside, the position of the convex column 31 corresponds to position of the slot 221, and the convex column 31 slightly protrudes from the outer surface of the outer sleeve 22. When the cartridge 3 is put into the outer sleeve 22, the convex column 31 slides along the slot 221, thereby ensuring the successful assembly and ensuring the cartridge 3 to be stable and not to swing in the outer sleeve 22.After the cartridge 3 is assembled, the bottom cap 24 is mounted. Then, the atomization assembly 2 is overturned entirely, so that the mouthpiece 21 is disposed at the upper end. Thus, the working state is ready.

In the second embodiment, the number of the slot 221 is two preferably, and the number of the convex column 31 is two too. That is, the slots 221 are defined on two lateral edges of the outer sleeve 22 symmetrically. Such arrangement has advantages that, no matter the cartridge 3 is put into or removed from the outer sleeve 22, the cartridge 3 can be moved by clamping the convex columns 31 with two fingers of a single hand respectively. Another purpose of the slot 221 lies in that the user can view the usage and consumption condition of the cartridge 3 through the slot 221, so as to replace the cartridge 3 in time and prevent dry-burning.

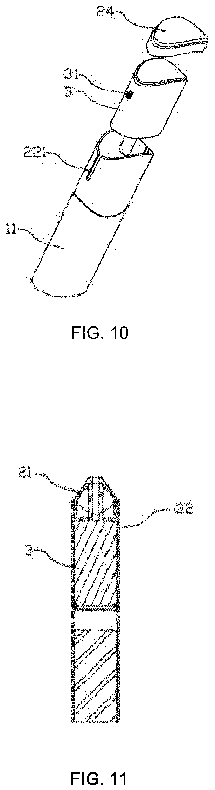

A third embodiment of the electronic cigarette is as shown in FIG. 11 to FIG. 12. The present embodiment has most of the structure the same as the first embodiment, with differences lying that the mouthpiece 21 and the outer sleeve 22 are in a detachable structure, the mouthpiece 21 has a tail end accommodated into a groove on the upper end of the outer sleeve 22, and the mouthpiece 21 and the outer sleeve 22 are in pluggable connection. Such arrangement has the purpose that the user can replace the mouthpiece with different shapes of mouthpieces according to the personal habit, or that the mouthpiece can be replaced separately when aged or accidentally damaged or contaminated, without the replacement of the outer sleeve 22, so that the cost is saved and the selectivity of the user is increased.

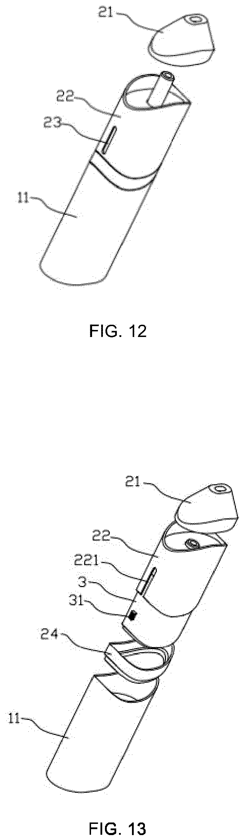

A fourth embodiment of the electronic cigarette is as shown in FIG. 13. The present embodiment has most of the structure the same as the first embodiment, with differences lying that the cartridge 3 and the outer sleeve 22 are in a detachable structure and the mouthpiece 21 and the outer sleeve 22 are in a detachable structure.

## Claims

1. An electronic cigarette, comprising a battery assembly (1) and an atomization assembly (2), the battery assembly (1) comprising a battery (12) and a housing (11), and the atomization assembly (2) comprising a mouthpiece (21), an outer sleeve (22) and a cartridge, wherein the atomization assembly (2) and the battery assembly (1) are in pluggable connection, the housing (11) defines a groove (13) in the upper part thereof, the outer sleeve (22) has a tail end accomodatable into the groove (13) in a working state, and the atomization assembly (2) is invertible and the mouthpiece (21) is accommodated into the groove (13) in a receiving state,
**characterized in that**:
the cartridge (3) and the outer sleeve (22) are in a detachable structure;
wherein the outer sleeve (22) defines a longitudinal slot (221) on a lateral edge thereof, the cartridge (3) is provided with a convex column (31) on the outside, the position of the convex column (31) corresponds to the position of the slot (221), and the convex column (31) protrudes from the outer surface of the outer sleeve (22).

2. The electronic cigarette according to claim 1, wherein the outer sleeve (22) has an outer surface matched with the housing (11) in shape and size.

3. The electronic cigarette according to claim 1, wherein the mouthpiece (21) and the outer sleeve (22) of the atomization assembly (2) are in an integrated structure or a detachable structure.

4. The electronic cigarette according to claim 1, wherein the number of the slot (221) is two, and the number of the convex column (31) is two too.

5. The electronic cigarette according to claim 1, wherein the outer sleeve (22) is provided with a bottom cap (24); the bottom cap (24) and the mouthpiece (21) are disposed at two opposite ends of the outer sleeve (22) respectively.

6. The electronic cigarette according to claim 1, wherein the outer sleeve (22) defines a longitudinal vision window (23).

## Patentansprüche

1. Elektronische Zigarette, die eine Batterieanordnung (1) und eine Zerstäubungsanordnung (2) umfasst, wobei die Batterieanordnung (1) eine Batterie (12) und ein Gehäuse (11) umfasst und die Zerstäubungsanordnung (2) ein Mundstück (21), eine äußere Hülse (22) und eine Patrone umfasst, wobei die Zerstäubungsanordnung (2) und die Batterieanordnung (1) in einer steckbaren Verbindung stehen, das Gehäuse (11) in seinem oberen Teil eine Nut (13) definiert, die äußere Hülse (22) ein hinteres Ende hat, das in einem Arbeitszustand in der Nut (13) untergebracht werden kann, und die Zerstäubungsanordnung (2) umkehrbar ist und das Mundstück (21) in einem Aufnahmezustand in der Nut (13) untergebracht ist, **dadurch gekennzeichnet, dass**:
die Patrone (3) und die äußere Hülse (22) in einer abnehmbaren Struktur sind;
wobei die äußere Hülse (22) einen Längsschlitz (221) an einem seitlichen Rand davon definiert, die Patrone (3) mit einer konvexen Säule (31) an der Außenseite versehen ist, die Position der konvexen Säule (31) der Position des Schlitzes (221) entspricht und die konvexe Säule (31) von der Außenfläche der äußeren Hülse (22) vorsteht.

2. Elektronische Zigarette nach Anspruch 1, wobei die äußere Hülse (22) eine Außenfläche aufweist, die in Form und Größe an das Gehäuse (11) angepasst ist.

3. Elektronische Zigarette nach Anspruch 1, wobei das Mundstück (21) und die äußere Hülse (22) der Zerstäubungseinheit (2) eine integrierte Struktur oder eine abnehmbare Struktur sind.

4. Elektronische Zigarette nach Anspruch 1, wobei die Anzahl des Schlitzes (221) zwei und die Anzahl der konvexen Säule (31) ebenfalls zwei ist.

5. Elektronische Zigarette nach Anspruch 1, wobei die äußere Hülse (22) mit einer Bodenkappe (24) versehen ist; die Bodenkappe (24) und das Mundstück (21) sind jeweils an zwei gegenüberliegenden Enden der äußeren Hülse (22) angeordnet.

6. Elektronische Zigarette nach Anspruch 1, wobei die äußere Hülse (22) ein Längssichtfenster (23) definiert.

## Revendications

1. Cigarette électronique, comprenant un ensemble de batterie (1) et un ensemble d'atomisation (2), l'ensemble de batterie (1) comprenant une batterie (12) et un logement (11), et l'ensemble d'atomisation (2) comprenant un embout (21), un manchon extérieur (22) et une cartouche, dans laquelle l'ensemble d'atomisation (2) et l'ensemble de batterie (1) sont en connexion enfichable, le boîtier (11) définit une rainure (13) dans sa partie supérieure, le manchon extérieur (22) a une extrémité de queue pouvant être logée dans la rainure (13) dans un état de travail, et l'ensemble de pulvérisation (2) est inversable et l'embout (21) est logé dans la rainure (13) dans un état de réception, **caractérisé en ce que**
la cartouche (3) et le manchon extérieur (22) sont dans une structure détachable ;
dans laquelle le manchon extérieur (22) définit une fente longitudinale (221) sur un bord latéral de celui-ci, la cartouche (3) est pourvue d'une colonne convexe (31) sur l'extérieur, la position de la colonne convexe (31) correspond à la position de la fente (221), et la colonne convexe (31) fait saillie de la surface extérieure du manchon extérieur (22).

2. Cigarette électronique selon la revendication 1, dans laquelle le manchon extérieur (22) a une surface extérieure dont la forme et la taille correspondent à celles du boîtier (11).

3. Cigarette électronique selon la revendication 1, dans laquelle l'embout (21) et le manchon extérieur (22) de l'ensemble d'atomisation (2) sont dans une structure intégrée ou une structure détachable.

4. Cigarette électronique selon la revendication 1, dans laquelle le nombre de fentes (221) est de deux, et le nombre de colonnes convexes (31) est également de deux.

5. Cigarette électronique selon la revendication 1, dans laquelle le manchon extérieur (22) est muni d'un capuchon inférieur (24) ; le capuchon inférieur (24) et l'embout (21) sont disposés respectivement à deux extrémités opposées du manchon extérieur (22).

6. Cigarette électronique selon la revendication 1, dans laquelle le manchon extérieur (22) définit une fenêtre de vision longitudinale (23).
